# EUROPÄISCHE PATENTSCHRIFT

(11) **EP 2 432 773 B1**
(45) Veröffentlichungstag und Bekanntmachung des Hinweises auf die Patenterteilung: **28.08.2013**
(21) Anmeldenummer: 10719340.1
(22) Anmeldetag: 11.05.2010
(51) Int. Cl.: C07D 309/12

(54) **Verfahren zur Herstellung von 2-substituierten Tetrahydropyranolen**
Process for producing 2-substituted tetrahydropyranoles
Procédé de préparation de tétrahydropyranols substitués en position 2

(30) Priorität: 19.05.2009 EP 09160665
(43) Veröffentlichungstag der Anmeldung: 28.03.2012
(73) Patentinhaber: BASF SE, 67056 Ludwigshafen (DE)
(72) Erfinder: GRALLA, Gabriele, 68161 Mannheim (DE); EBEL, Klaus, 68623 Lampertheim (DE); GRIESBACH, Ulrich, 68305 Mannheim (DE); PELZER, Ralf, 37699 Fürstenberg (DE); BOTZEM, Jörg, 67117 Limburgerhof (DE)
(86) Internationale Anmeldenummer: PCT/EP2010/056403
(87) Internationale Veröffentlichungsnummer: WO 2010/133473

(56) Entgegenhaltungen:
- EP-A1- 1 493 737
- DATABASE WPI Week 200755 Thomson Scientific, London, GB; AN 2007-564955 XP002597081 -& JP 2007 154069 A (HASEGAWA CO LTD) 21. Juni 2007 (2007-06-21)
- DATABASE WPI Week 198206 Thomson Scientific, London, GB; AN 1982-11549E XP002597059 -& SU 825 528 A1 (AS ARMN ORG CHEM) 30. April 1981 (1981-04-30)
- A. A. GEVORKYAN ET AL.: "Mechanism of cycloalkylation of allylcarbinols with aldehydes and ketones" CHEMISTRY OF HETEROCYCLIC COMPOUNDS, Nr. 12, 1982, Seiten 1240-1242, XP002597060

## Beschreibung

Die vorliegende Erfindung betrifft ein Verfahren zur Herstellung von 2-sustituierten 4-Hydroxy-4-methyl-tetrahydropyranolen durch Umsetzung von 3-Methylbut-3-en-1-ol (Isoprenol) mit den entsprechenden Aldehyden in Gegenwart eines stark sauren Kationenaustauschers. Speziell betrifft die vorliegende Erfindung ein entsprechendes Verfahren zur Herstellung von 2-Isobutyl-4-hydroxy-4-methyl-tetrahydropyran durch Umsetzung von Isoprenol mit Isovaleraldehyd.

In Tetrahedron Letters No. 51, Seiten 4507-4508,1970 wird die Reaktion von 3-Alken-1-olen mit Aldehyden und deren Anwendung zur Herstellung der Aromachemikalien Rosenoxid bzw. Dihydrorosenoxid beschrieben. Dabei wird auch die Umsetzung von 3-Methylbutanal mit Isoprenol unter sauren Bedingungen erwähnt.

In Chemistry of Heterocyclic Compounds, Seiten 1107 - 1109, 1990 wird die Kondensation von Isoprenol mit verschiedenen Aldehyden und Ketonen zu den entsprechenden Di- und Tetrahydropyranen in Gegenwart von Kieselgel oder Al₂O₃ unter lösungsmittelfreien Bedingungen beschrieben. Pyranole werden dabei nur in geringem Ausmaß bei Verwendung von Al₂O₃ erhalten.

Die SU 825 528 offenbart ein Verfahren zur Herstellung von Di- bzw. Tetrahydropyranen und Tetrahydropyranolen durch Umsetzung von 2-Methyl-buten-1-ol-4 (Isoprenol) mit Aldehyden oder Ketonen in Gegenwart eines sauren Katalysators, wobei der saure Katalysator in einer Menge von 0,0001 bis 0,01 Gew.-% bezogen auf die Menge an Isoprenol eingesetzt wird, und die Umsetzung bei einer Temperatur von 0 bis 25°C in einem organischen Lösungsmittel durchgeführt wird. Als Katalysatoren werden der Ionenaustauscherharz KU-2 (sulfoniertes Polystyrolharz), para-Toluolsulfonsäure, Schwefelsäure, Phosphorsäure oder Perchlorsäure genannt. Beispielhaft wird u.a. die Umsetzung von Isoprenol mit Isobutyraldehyd in Gegenwart von KU-2 beschrieben.

EP 1 493 737 A1 offenbart ein Verfahren zur Herstellung von Gemischen von ethylenisch ungesättigten 4-Methyl- bzw. 4-Methylenpyranen und den entsprechenden 4-Hydroxypyranen durch Umsetzung der entsprechenden Aldehyde mit Isoprenol, wobei die Umsetzung in einem Reaktionssystem initiiert wird, in dem das molare Verhältnis von Aldehyd zu Isoprenol größer als 1 ist, d.h. der Aldehyd im Überschuss eingesetzt wird. Darüber hinaus offenbart das Dokument die anschließende Dehydrierung der genannten Gemische zu den gewünschten ethylenisch ungesättigten Pyranen. Als geeignete Katalysatoren für den ersten Reaktionsschritt werden Mineralsäuren wie Salzsäure oder Schwefelsäure, bevorzugt jedoch Methansulfonsäure oder para-Toluolsulfonsäure genannt.

EP 1 516 879 A1 offenbart ein Verfahren zur Herstellung von ethylenisch ungesättigten 4-Methyl- bzw. 4-Methylenpyranen durch Umsetzung eines entsprechenden Aldehyds mit Isoprenol unter dehydrierenden Bedingungen, wobei die Wassermenge im Reaktor bis zu 0,25 Gew.-% beträgt, während der Umsatz der im Unterschuss eingesetzten Ausgangsverbindung weniger als 50% beträgt. Als hierfür geeignete Katalysatoren werden ebenfalls Mineralsäuren wie Salzsäure oder Schwefelsäure, bevorzugt jedoch Methansulfonsäure oder para-Toluolsulfonsäure genannt.

JP 2007-154069 betrifft 2-substituierte 4-Hydroxy-4-Methyl-tetrahydropyranole mit einem Gehalt des cis-Diastereomeren von 70 bis 95 Gew.-%. Das Dokument offenbart darüber hinaus ein Verfahren zur Herstellung derselben, durch Umsetzung von Isoprenol mit einem entsprechenden Aldehyd in Gegenwart einer wässrigen Lösung eines sauren Katalysators. Dabei muss die Umsetzung bei einer Konzentration der wässrigen Katalysatorlösung entweder im Bereich von 1 bis 10 Gew.-% bei einer Temperatur von 0 bis 100°C durchgeführt werden oder im Bereich von 10 Gew.-% oder darüber bei einer Temperatur von 0 bis 30°C. Als mögliche saure Katalysatoren werden allgemein auch lonentauscherharze genannt.

Ausgehend von diesem Stand der Technik bestand die Aufgabe der vorliegenden Erfindung in der Bereitstellung eines Verfahrens zur Herstellung von 2-substituierten 4-Hydroxy-4-methyl-tetrahydropyranen, speziell 2-Isobutyl-4-hydroxy-4-methyl-tetrahydropyran, das die gewünschten Verbindungen möglichst
- ausgehend von gut verfügbaren, wohlfeilen Edukten,
- unter Verwendung gut verfügbarer, wohlfeiler Reagenzien
- in verfahrenstechnisch vorteilhafter Weise,
- im technischen Maßstab,
- in hoher Ausbeute,
- in hohem Diastereomerenüberschuss,
- mit möglichst geringer Bildung unerwünschter und zu entsorgender Nebenprodukte und
- mit möglicht vorteilhaften geruchlichen Eigenschaften

### zugänglich macht.

Die Aufgabe wurde in überraschender Weise erfindungsgemäß gelöst durch die Bereitstellung eines Verfahrens zur Herstellung von 2-substituierten 4-Hydroxy-4-methyl-tetrahydropyranen der Formel (I) 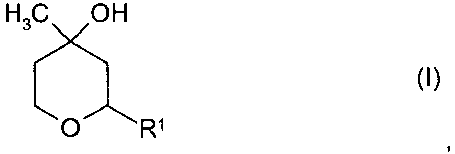 wobei der Rest
- R¹: einen geradkettigen oder verzweigten Alkyl- oder Alkenylrest mit 1 bis 12 Koh-lenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen bedeutet,
umfassend die Umsetzung von 3-Methylbut-3-en-1-ol der Formel (II) 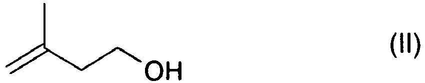 mit einem Aldehyd der Formel (III)

R¹-CHO (III),

wobei der Rest R¹ die gleiche Bedeutung wie in Formel (I) hat und
wobei man die Umsetzung in Gegenwart von Wasser und in Gegenwart eines stark sauren Kationenaustauschers durchführt.

Als Ausgangsstoffe zur Durchführung des erfindungsgemäßen Verfahrens dienen 3-Methylbut-3-en-1-ol (isoprenol) der Formel (II), 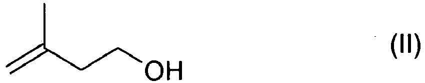 das nach bekannten Verfahren aus Isobuten und Formaldehyd in jedem Maßstab gut zugänglich und kommerziell gut verfügbar ist. An die Reinheit, Qualität oder Herstellverfahren des erfindungsgemäß einzusetzenden Isoprenols sind keine besonderen Anforderungen zu stellen. Es kann in handelsüblicher Qualität und Reinheit mit gutem Erfolg als Ausgangsstoff im Rahmen des erfindungsgemäßen Verfahrens eingesetzt werden. Bevorzugt setzt man Isoprenol ein, das eine Reinheit von 90 Gew.-% oder darüber hat, besonders bevorzugt solches mit einer Reinheit von 95 bis 100 Gew.-% und ganz besonders bevorzugt solches mit einer Reinheit von 97 bis 99,9 Gew% oder noch mehr bevorzugt 98 bis 99,8 Gew.-%.

Als weiterer Ausgangsstoff zur Durchführung des erfindungssgemäßen Verfahrens dient ein Aldehyd der Formel (III)

R¹-CHO (III),

wobei der Rest R¹ für einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen stehen kann. Dabei ist unter dem Begriff Alkenylrest ein solcher Kohlenwaserstoffrest zu verstehen, der neben Einfachbindungen noch eine oder mehrere, bevorzugt 1 bis 3, besonders bevorzugt 1 oder 2 und ganz besonders bevorzugt eine ethylenische Doppelbindung aufweist.

Unter einem Alkylsubstituenten ist bevorzugt ein solcher zu verstehen der 1 bis 6 Kohlenstoffatome aufweist wie beispielsweise Methyl, Ethyl, n-Propyl, iso-Propyl, n-Butyl, sec-Butyl, tert.-Butyl, n-Pentyl oder n-Hexyl, bevorzugt Methyl, Ethyl, n-Propyl, iso-Propyl, Isobutyl.

Unter einem Alkoxysubstituenten ist bevorzugt ein solcher zu verstehen der 1 bis 6 Kohlenstoffatome, besonders bevorzugt 1 bis 3 Kohlenstoffatome aufweist wie beispielsweise Methoxy, Ethoxy, n-Propyloxy, iso-Propyloxy.

Erfindungsgemäß bevorzugte Aldehyde der Formel (III) sind solche, bei denen der Rest R¹ für einen geradkettigen oder verzweigten Alkyl- oder Alkenylrest mit 1 bis 12 Kohlenstoffatomen, oder einen gegebenenfalls Alkyl- und/oder Alkoxy-substituierten Arylrest mit insgesamt 6 bis 12 Kohlenstoffatomen steht. Erfindungsgemäß ganz besonders bevorzugte Aldehyde der Formel (III) sind solche, bei denen der Rest R¹ für einen geradkettigen oder verzweigten Alkyl- oder Alkenylrest mit 1 bis 12 Kohlenstoffatomen oder bevorzugt mit 1 bis 6 Kohlenstoffatomen, oder für einen Arylrest mit insgesamt 6 Kohlenstoffatomen, d.h. für Phenyl steht. Insbesonders bevorzugte Aldehyde der Formel (III) sind solche, bei denen der Rest R¹ für einen geradkettigen oder verzweigten Alkyl- oder Alkenylrest mit 1 bis 12 Kohlenstoffatomen, ganz besonders bevorzugt mit 1 bis 6 Kohlenstoffatomen steht. Erfindungsgemäß bevorzugte Bedeutungen für den Rest R¹ sind somit beispielsweise Methyl, Ethyl, n-Propyl, iso-Propyl, n-Butyl, iso-Butyl, n-Pentyl, n-Hexyl, n-Heptyl, bevorzugt Methyl, Ethyl, n-Propyl, iso-Propyl, n-Butyl, iso-Butyl, ganz besonders bevorzugt iso-Butyl. Als dementsprechend erfindungsgemäß bevorzugt einzusetzende Aldehyde der Formel (III) seien genannt: Acetaldehyd, Valeraldehyd, Isovaleraldehyd, Pentanal, Hexanal, Heptanal, Benzaldehyd, Citral, Citronellal. Erfindungsgemäß ganz besonders bevorzugt einzusetzende Aldehyde der Formel (III) sind somit Isovaleraldehyd und Benzaldehyd, insbesondere Isovaleraldehyd.

Die vorliegende Erfindung betrifft daher im Rahmen einer bevorzugten Ausführungsform ein Verfahren zur Herstellung von 2-Iso-Butyl-4-Hydroxy-4-methyl-tetrahydropyran der Formel (la) 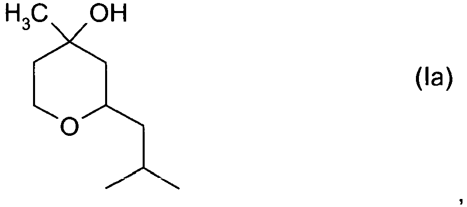 umfassend die Umsetzung von 3-Methylbut-3-en-1-ol der Formel (II) mit Isovaleraldehyd der Formel (IIIa) 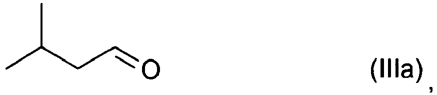 wobei man die Umsetzung in Gegenwart von Wasser und in Gegenwart eines stark sauren Kationenaustauschers durchführt.

Die vorliegende Erfindung betrifft im Rahmen einer weiteren, ebenfalls bevorzugten Ausführungsform ein Verfahren zur Herstellung von 2-Phenyl-4-hydroxy-4-methyl-tetrahydropyran der Formel (la') 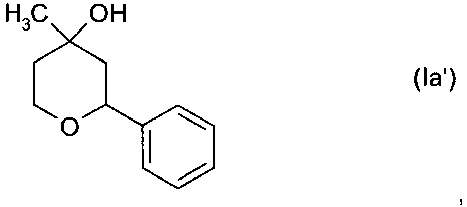 umfassend die Umsetzung von 3-Methylbut-3-en-1-ol der Formel (II) mit Benzaldehyd, wobei man die Umsetzung in Gegenwart von Wasser und in Gegenwart eines stark sauren Kationenaustauschers durchführt.

Die im Rahmen des erfindungsgemäßen Verfahrens einzusetzenden Ausgangsstoffe Isoprenol und der jeweils gewählte Aldehyd der Formel (III) können in unterschiedlichen Mengenverhältnissen miteinander umgesetzt werden. So ist es möglich eine der beiden Ausgangsstoffe im Überschuss einzusetzen, wobei die Höhe des gewählten Überschusses sich in verfahrenstechnisch und wirtschaftlich vorteilhaften Grenzen bewegen sollte, ansonsten im Prinzip jedoch frei gewählt werden kann. Der Stöchiometrie der erfindungsgemäßen Umsetzung von Isoprenol mit dem gewählten Aldehyd der Formel (III) folgend, werden Isoprenol und der Aldehyd der Formel (III), bevorzugt Isovaleraldehyd, in einem molaren Verhältnis im Bereich von 1 zu 2 bis 2 zu 1 eingesetzt, entsprechend einem doppelten molaren Überschuss einer der Ausgangsstoffe. Im Rahmen einer bevorzugten Ausführungsform führt man das erfindungsgemäße Verfahren so durch, dass man Isoprenol und den Aldehyd der Formel (III) in einem molaren Verhältnis von 0,7 zu 1 bis 2 zu 1 einsetzt. Besonders bevorzugt führt man das erfindungsgemäße Verfahren so durch, dass man Isoprenol und den Aldehyd der Formel (III) in einem molaren Verhältnis von 1 zu 1 bis 2 zu 1 einsetzt. Ganz besonders bevorzugt führt man das erfindungsgemäße Verfahren so durch, dass man Isoprenol und den Aldehyd der Formel (III) in einem molaren Verhältnis von 1 zu 1 bis 1,5 zu 1 einsetzt.

Die im Rahmen des erfindungsgemäßen Verfahren zur Herstellung der 2-substituierten 4-Hydroxy-4-methyl-tetrahydropyranen der Formel (I), bevorzugt zur Herstellung von 2-Iso-Butyl-4-Hydroxy-4-methyl-tetrahydropyran der Formel (la) durchzuführende Umsetzung von Isoprenol mit dem gewählten Aldehyd der Formel (III), bevorzugt mit Isovaleraldehyd, wird in Gegenwart von Wasser durchgeführt. Dies bedeutet, dass dem Reaktionsgemisch neben Isoprenol, dem Aldehyd der Formel (III) und dem gewählten stark sauren Kationenaustauscher auch Wasser zugesetzt wird. Zusätzlich kann das Reaktionsgemisch noch geringe Mengen Wasser enthalten, das durch die möglicherweise als unerwünschte Nebenreaktion erfolgende Dehydratisierung des gewünschten Verfahrensproduktes der Formel (I) freigesetzt werden kann.

Üblicherweise führt man die Umsetzung des Isoprenols mit dem gewählten Aldehyd der Formel (III) in Gegenwart von etwa mindestens 10 mol-% Wasser durch, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht.

Oberhalb des angegebenen Wertes kann die Menge an Wasser frei gewählt werden und ist, wenn überhaupt, nur durch verfahrenstechnische oder ökonomische Aspekte begrenzt und kann durchaus in großem, beispielsweise in 10- bis 100-fachem Überschuss oder auch darüber eingesetzt werden. Vorzugsweise bereitet man ein Gemisch aus Isoprenol und dem gewählten Aldehyd der Formel (III), vorzugsweise Isovaleraldehyd, mit der gewählten Menge Wasser, so dass das zugegebene Wasser in dem Gemisch aus Isoprenol und dem gewählten Aldehyd gelöst bleibt d.h. kein zweiphasiges System vorliegt.

Üblicherweise setzt man im Rahmen des erfindungsgemäßen Verfahrens die Ausgangsstoffe Isoprenol und den gewählten Aldehyd der Formel (III) in Gegenwart von mindestens 25 mol-%, bevorzugt von mindestens 50 mol-%, noch mehr bevorzugt von mindestens 75 und noch mehr bevorzugt von mindestens 90 bis etwa 1000 mol-% Wasser um, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht.

Im Rahmen einer bevorzugten Ausführungsform führt man die erfindungsgemäß durchzuführende Umsetzung so durch, dass man sie in Gegenwart einer mindestens äquimolaren Menge an Wasser durchführt, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht. Demzufolge führt man die erfindungsgemäße Umsetzung von Isoprenol mit dem gewählten Aldehyd der Formel (III) bevorzugt in Gegenwart von 100 bis 250 mol-%, besonders bevorzugt 100 bis 230 mol-%, noch mehr bevorzugt 100 bis 200 mol-% und am meisten bevorzugt in Gegenwart von 100 bis 180 mol-% Wasser durch, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht.

Die genannten Ausgangsstoffe, d.h. Isoprenol und der jeweils gewählte Aldehyd und das in der vorstehenden Menge einzusetzende Wasser können in beliebiger Reihenfolge miteinander in Kontakt gebracht bzw. gemischt werden. Üblicherweise bereitet man ein Gemisch aus Isoprenol und dem gewählten Aldehyd der Formel (III) mit der gewählten Menge Wasser und setzt dieses Gemisch im Rahmen der erfindungsgemäß durchzuführenden Umsetzung ein.

Die im Rahmen des erfindungsgemäßen Verfahrens zur Herstellung der gewünschten 2-substituierten 4-Hydroxy-4-methyl-tetrahydropyranen der Formel (I) durchzuführende Umsetzung von Isoprenol mit dem gewählten Aldehyd der Formel (III) wird auch in Gegenwart eines stark sauren Kationenaustauschers durchgeführt. Unter dem Begriff stark saurer Kationenaustauscher sind dabei im Rahmen der vorliegenden Erfindung solche Kationenaustauscher in der H⁺-Form zu verstehen, die stark saure Gruppen, in der Regel Sulfonsäuregruppen, aufweisen, deren Matrix gelförmig bzw. makroporös sein kann.

Eine bevorzugte Ausführungsform des erfindungsgemäßen Verfahrens ist dementsprechend dadurch gekennzeichnet, dass man einen stark sauren, Sulfonsäuregruppen aufweisenden bzw. umfassenden Kationenaustauscher einsetzt.

Stark saure Kationenaustauscher sind insbesondere lonenaustauscherharze in der H(+)-Form. Als solche kommen beispielsweise in Betracht:
- stark saure Ionenaustauscher (wie z.B. Amberlyst, Amberlite, Dowex, Lewatit, Purolite, Serdolit), die auf Polystyrol basieren, und die Copolymere aus Styrol und Divinylbenzol als Trägermatrix mit Sulfonsäuregruppen in H(+)-Form enthalten,
- mit Sulfonsäuregruppen (-SO₃H) funktionalisierte lonenaustauschergruppen.

Die lonenaustauscher unterscheiden sich im Aufbau ihrer Polymergerüste, und man unterscheidet gelförmige und makroporöse Harze. Die stark sauren lonentauscherharze werden in der Regel mit Salzsäure und / oder Schwefelsäure regeneriert.

Nafion^{®} ist die Bezeichnung der Firma Dupont für perfluorierte polymere lonenaustauscherharze. Es handelt sich dabei um perfluorierte lonenaustauschmaterialien bestehend aus Fluorkohlenstoff-Basisketten und perfluorierten Seitenketten, die Sulfonsäuregruppen enthalten. Die Harze werden durch eine Copolymerisation von perfluorierten, endständig ungesättigten und mit Sulfonylfluorid funktionalisierten Ethoxylaten mit Perfluorethen hergestellt. Nafion^{®} zählt zu den gelartigen lonenaustauscherharzen. Als Beispiel für ein solches perfluoriertes polymeres lonenaustauscherharz sein Nafion^{®} NR-50 genannt.

Eine besonders bevorzugte Ausführungsform des erfindungsgemässen Verfahrens ist dadurch gekennzeichnet, dass man mindestens einen stark sauren Kationenaustauscher in der H(+)-Form einsetzt, wobei der lonenaustauscher ein Sulfonsäuregruppen aufweisendes Polymergerüst enthält und entweder gelförmig ist oder makroporöse Harze enthält.

Eine ganz besonders bevorzugte Ausführungsform des erfindungsgemässen Verfahrens ist dadurch gekennzeichnet, dass der Ionenaustauscher auf einem Polystyrolgerüst mit Sulfonsäuregruppen oder auf einem perfluorierten lonenaustauscherharz mit Sulfonsäuregruppen basiert.

Die kommerziell verfügbaren stark sauren Kationenaustauscher sind unter den Handelsnamen Lewatit® (Lanxess), Purolite® (The Purolite Company), Dowex® (Dow Chemical Company), Amberlite® (Rohm and Haas Company), Amberlyst™ (Rohm and Haas Company) bekannt.

Als erfindungsgemäß bevorzugte stark saure Kationenaustauscher seien beispielsweise genannt: Lewatit^{®} K 1221, Lewatif^{®} K 1461, Lewatit^{®} K 2431, Lewatit^{®} K 2620, Lewatit^{®} K 2621, Lewatit^{®} K 2629, Lewatit^{®} K 2649, Amberlite^{®} IR 120, Amberlyst™ 131, Amberlst™ 15, Amberlyst™ 31, Amberlyst™ 35, Amberlyst™ 36, Amberlyst™ 39, Amberlyst™ 46, Amberlyst™ 70, Purolite^{®} SGC650, Purolite^{®} C100H, Purolite^{®} C150H, Dowex^{®} 50X8, Serdolit^{®} rot und Nafion^{®} NR-50.

Im Rahmen einer bevorzugten Ausführungsform führt man die erfindungsgemäß durchzuführende Umsetzung von Isoprenol mit dem gewählten Aldehyd der Formel (III) in Gegenwart mindestens eines stark sauren Kationenaustauschers durch, der ausgewählt ist aus der Gruppe der Kationenaustauscher umfassend Lewatit^{®} K 1221, Lewatit^{®} K 2629, Amberlyst™ 131, Purolite^{®} SGC650, Purolite^{®} C100H, Purolite^{®} C150H, Amberlite^{®} IR 120 und Dowex^{®} 50X8.

Erfindunggsgemäß insbesondere bevorzugte stark saure Kationentauscher sind die Kationenaustauscher Amberlyst™ 131 und/oder Lewatit^{®} K 1221.

Ein erfindungsgemäß ganz besonders bevorzugter stark saurer Kationenaustauscher ist Amberlyst™ 131, der wie die anderen genannten Kationenaustauscher kommerziell verfügbar ist.

Zur Durchführung der erfindungsgemäßen Umsetzung von Isoprenol mit dem Aldehyd der Formel (III) bringt man die genannten Ausgangsstoffe und die gewählte Menge Wasser, vorzugsweise in Form eines Gemisches, mit dem gewählten stark sauren Kationenaustauscher in Kontakt. Die Menge an einzusetzendem Kationenaustauscher ist nicht kritisch und kann unter Berücksichtigung des wirtschaftlichen und verfahrenstechnischen Aspektes in breiten Grenzen frei gewählt werden. Die Umsetzung kann dementsprechend sowohl in Gegenwart katalytischer Mengen als auch in Gegenwart großer Überschüsse des gewählten stark sauren Kationenaustauschers durchgeführt werden. Üblicherweise setzt man den gewählten Kationentauscher in einer Menge von etwa 5 bis etwa 40 Gew.-%, bevorzugte in einer Menge von etwa 20 bis etwa 40 Gew.-% und besonders bevorzugt in einer Menge von etwa 20 bis etwa 30 Gew.-%, jeweils bezogen auf die Summe an eingesetztem Isoprenol und Aldehyd der Formel (III), ein. Dabei beziehen sich die Angaben auf den gebrauchsfertigen Kationenaustauscher, der in der Regel mit Wasser vorbehandelt wird und dementsprechend Mengen von bis zu etwa 70 Gew.%, bevorzugt von etwa 30 bis etwa 65 Gew-% und besonders bevorzugt von etwa 40 bis etwa 65 Gew.-% Wasser umfassen kann. Insbesondere bei diskontinuierlicher Verfahrensführung kann sich daher ein darüber hinausgehender Wasserzusatz bei der Durchführung des erfindungsgemäßen Verfahrens erübrigen.

Die genannten stark sauren Kationenaustauscher können sowohl einzeln oder auch in Form von Gemischen untereinander im Rahmen des erfindungsgemäßen Verfahrens eingesetzt werden.

Die erfindungsgemäß durchzuführende Umsetzung kann wahlweise auch in Gegenwart eines unter den Reaktionsbedingungen inerten Lösungsmittels wie beispielsweise tert-Butylmethylether, Cyclohexan, Toluol, Hexan oder Xylol durchgeführt werden. Die genannten Lösungsmittel können alleine oder in Form von Gemischen untereinander eingesetzt werden. Im Rahmen einer bevorzugten Ausführungsform des erfindungsgemäßen Verfahrens führt man die Umsetzung von Isoprenol mit dem gewählten Aldehyd der Formel (III) ohne Zusatz eines organischen Lösungsmittels durch.

Die erfindungsgemäß durchzuführende Umsetzung von Isoprenol mit dem gewählten Aldehyd der Formel (III) in Gegenwart von Wasser und in Gegenwart eines stark sauren Kationentauschers wird üblicherweise bei einer Temperatur im Bereich von 0 bis 60°C, bevorzugt bei einer Temperatur im Bereich von 20 bis 60°C und besonders bevorzugt bei einer Temperatur im Bereich von 20 bis 50°C durchgeführt, wobei sich die Temperatur auf die des Reaktionsgemisches bezieht.

Die erfindungsgemäß durchzuführende Umsetzung kann wahlweise diskontinuierlich oder kontinuierlich durchgeführt werden. Dabei kann man beispielsweise im diskontinuierlichen Fall die Umsetzung so vornehmen, dass man ein Gemisch von Isoprenol, dem gewählten Aldehyd der Formel (III) und Wasser in einem geeigneten Reaktionsgefäß vorlegt und den stark sauren Kationenaustauscher zugibt. Nach Abschluss der Reaktion kann dann der Kationenaustauscher durch geeignete Trennverfahren, vorzugsweise durch Filtration oder auch durch Zentrifugation, vom erhaltenen Reaktionsgemisch abgetrennt werden. Die Reihenfolge des Inkontaktbringens der einzelnen Reaktionskomponenten ist nicht kritisch und kann nach Maßgabe der jeweiligen verfahrenstechnischen Ausgestaltung variiert werden.

Im Rahmen einer bevorzugten Ausführungsform führt man die erfindungsgemäß durchzuführende Umsetzung von Isoprenol mit dem gewählten Aldehyd der Formel (III) kontinuierlich durch. Dazu kann beispielsweise eine Mischung der umzusetzenden Ausgangsstoffe Isoprenol und Aldehyd der Formel (III) mit Wasser bereitet werden und diese Mischung kontinuierlich mit einem stark sauren Kationentauscher in Kontakt gebracht werden. Dazu kann der gewählte Kationenaustauscher beispielsweise in einen geeigneten Durchflussreaktor, beispielsweise einen Rührreaktor mit Zu- und Ablauf oder einen Rohrreaktor eingebracht werden und die Ausgangsstoffe und das Wasser in diesen kontinuierlich ausgetragen werden und das Reaktionsgemisch kontinuierlich ausgetragen werden. Dabei können die Ausgangsstoffe und das Wasser wahlweise als Einzelkomponenten oder auch in Form eines wie vorstehend beschriebenen Gemisches in den Durchflussreaktor eingetragen werden.

Eine bevorzugte Ausführungsform des erfindungsgemäßen Verfahrens betrifft dementsprechend ein kontinuierliches Verfahren zur Herstellung von 2-substituierten 4-Hydroxy-4-methyl-tetrahydropyranen der Formel (I) umfassend die Schritte
a. Bereitstellen eines den gewählten stark sauren Kationenaustauscher enthaltenden Durchflussreaktors;
b. kontinuierliches Eintragen von Isoprenol, dem Aldehyd der Formel (III) sowie Wasser in den Durchflussreaktor;
c. kontinuierliches Inkontaktbringen von Isoprenol, dem Aldehyd der Formel (I-II) sowie Wasser mit dem stark sauren Kationenaustauscher im Durchflussreaktor unter Erhalt eines die gewünschten 2-substituierten 4-Hydroxy-4-methyl-tetrahydropyrane umfassenden Reaktionsgemisches und
d. kontinuierliches Austragen des Reaktionsgemisches aus dem Durchflussreaktor.

Der gewählte stark saure Kationenaustauscher kann dabei sowohl in Form einer losen Schüttung als auch in Form eines Festbettes im vorstehend genannten Durchflussreaktor vorliegen.

Es ist auch möglich die erfindungsgemäß durchzuführend Umsetzung von Isoprenol mit dem Aldehyd der Formel (III) in einer Kaskade von mehreren, beispielsweise 2 oder 3 hintereinander geschalteten Durchflussreaktoren durchzuführen, wobei die einzelnen Durchflussreaktoren auch mit verschiedenen stark sauren Kationenaustauschern befüllt sein können und im Fall des Einsatzes von Rohrreaktoren, diese sowohl in Sumpfals auch in Rieselfahrweise betrieben werden können. Darüber hinaus kann das aus dem gewählten Durchflussreaktor ausgetragene Reaktionsgemisch gewünschtenfalls auch teilweise wieder in die kontinuierlichbetriebene Umsetzung zurückgeführt werden.

Das erfindungsgemäße Verfahren ermöglicht die Herstellung von 2-substituierten 4-Hydroxy-4-methyl-tetrahydropyranen der Formel (I), speziell die Herstellung von 2-Iso-Butyl-4-Hydroxy-4-methyl-tetrahydropyranen der Formel (I). Diese fallen üblicherweise in Form von Reaktionsgemischen an, die neben den gewünschten Zielverbindungen, noch Reste der eingesetzten Ausgangsstoffe, das eingesetzte Wasser sowie möglicherweise in geringem Ausmaß auch die dehydratisierten Nebenprodukte der Formeln (IVa), (IVb) und/oder (IVc) 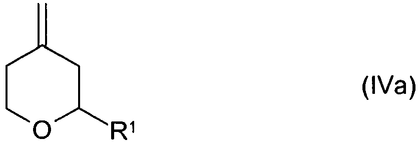 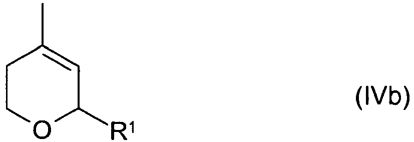 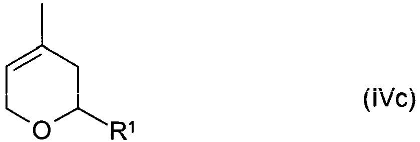 enthalten können. Das erfindungsgemäße Verfahren ermöglicht die Herstellung der gewünschten Hydroxypyrane der Formel (I) bzw. bevorzugt des 2-Iso-butyl-4-hydroxy-4-methyltetrahydropyrans der Formel (la) in hoher Ausbeute und hoher Reinheit, wobei die unerwünschten Dehydratisierungsprodukte der Formeln (IVa bis (IVc) wenn über-haupt, nur in untergeordnetem Ausmaß anfallen.

Als weitere mögliche Nebenprodukte seien die Acetale der Formel (V) 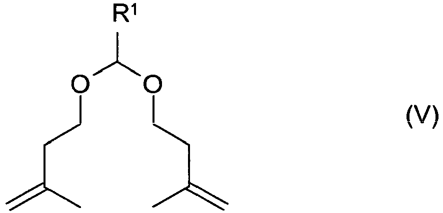 sowie die 1,3-Dioxane der Formel (VI) 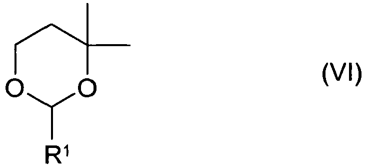 genannt, wobei im erfindungsgemäß bevorzugten Fall der Umsetzung von Isoprenol mit Isovaleraldehyd der Rest R¹ jeweils Iso-Butyl bedeutet (entsprechend den Verbindungen der Formeln (Va) bzw. (VIa). Diese Nebenprodukte können, genauso wie die nicht umgesetzten bzw. im Überschuss eingesetzten Ausgangsverbindungen in vorteilhafter Weise wieder in die Reaktion zurückgeführt werden.

Die erfindungsgemäß erhaltenen Reaktionsgemische bestehen typischerweise zu etwa 50 bis zu etwa 90 Gew.-%, häufig zu etwa 60 bis zu etwa 80 Gew.-% aus den gewünschten 2-substituierten 4-Hydroxy-4-methyl-tetrahydropyranen der Formel (I) und nur bis zu etwa 20 Gew.-%, bevorzugt nur bis zu etwa 15 Gew.-% und besonders bevorzugt nur bis zu 10 Gew.-% aus den Dehydratisierungsprodukten der Formeln (IVa) bis (IVc), jeweils bezogen auf das Gesamtgewicht des erhaltenen Rohproduktes und im übrigen aus den nicht umgesetzten bzw. im Überschuss eingesetzten Ausgangsstoffen sowie den anderen genannten Nebenprodukten.

Die als Rohprodukt erhaltenen Stoffgemische lassen sich gut nach dem Fachmann bekannten Verfahren, insbesondere durch Destillation bzw. Rektifikation, weiter aufreinigen. Auf diese Weise erhält man das jeweils gewünschte 2-substituierte 4-Hydroxy-4-methyl-tetrahydropyran der Formel (I), insbesondere bei Einsatz von Isoprenol und Isovaleraldehyd das gewünschte 2-Iso-butyl-4-hydroxy-4-methyltetrahydropyran der Formel (la) in einer Reinheit von über 95 Gew.-% oder bevorzugt von 97 bis 99,9 Gew.-% oder besonders bevorzugt von 98 bis 99,8 Gew-% , d.h. in einer Qualität, wie sie beispielsweise für den Einsatz als Aromachemikalie erforderlich ist.

Eine bevorzugte Ausführungsform des erfindungsgemäßen Verfahrens betrifft die Herstellung von 2-substituierten 4-Hydroxy-4-methyl-tetrahydropyranen in Form von Gemischen der cis-Diastereomeren der Formel (Ib) 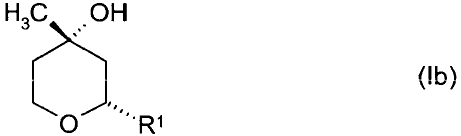 und der trans-Diastereomeren der Formel (Ic) 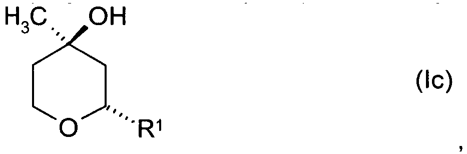 wobei das Diastereomerenverhältnis des cis-Diasteromeren der Formel (Ib) zum trans-Diastereomern der Formel (Ic) 65 zu 35 bis 95 zu 5, bevorzugt 70 zu 30 bis 85 zu 15 beträgt, und R¹ die weiter oben angegebenen Bedeutungen hat.

Insbesondere für die erfindungsgemäß bevorzugte Umsetzung von Isoprenol mit Isovaleraldehyd erhält man im Rahmen des erfindungsgemäßen Verfahrens 2-Isobutyl-4-hydroxy-4-methyl-tetrahydropyran in Form von Gemischen des cis-Diastereomeren der Formel (Id) 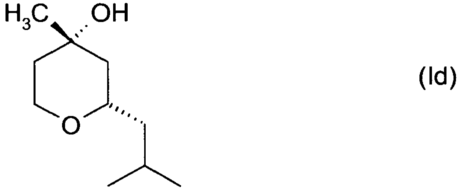 und der trans-Diastereomeren der Formel (le) 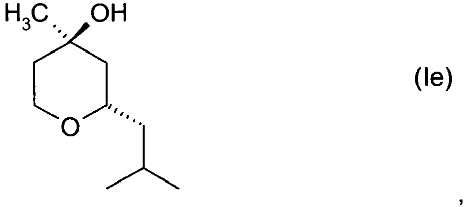 wobei das Diastereomerenverhältnis des cis-Diasteromeren der Formel (Id) zum trans-Diastereomeren der Formel (Ie) 65 zu 35 bis 95 zu 5, bevorzugt 70 zu 30 bis 85 zu 15 beträgt. Derartige Gemische eignen sich aufgrund ihrer besonderen geruchlichen Eigenschaften in besonderem Maße zur Verwendung als Aromachemikalien, beispielsweise als Komponente mit Maiglöckchenduft zur Herstellung von Riechstoffkompositionen.

Die folgenden Beispiele dienen der Erläuterung der Erfindung:
Gaschromatographische Analysen wurden nach der folgenden Methode durchgeführt: 30 m DB-WAX, ID.: 0,32 mm, FD.: 0,25 µm; 50°C, 3°C/min - 170 °C, 20°C/min auf 230 °C - 17 min; Inj. 200°C, Det. 280°C, t_{R} = min; t_{R} (Isovaleraldehyd): 4,1; t_{R} (Dihydropyran-Isomere der Formeln (IVa) bis (IVc)): 10,0; 11,8; 12,3; t_{R} (Isoprenol): 10,6;
t_{R} (1,3-Dioxan (Va)): 12,1; t_{R} (Acetal (VIa)): 24,1; t_{R} (trans-Pyranol der Formel (le)): 28,2; t_{R} (cis-Pyranol der Formel (Id)): 29,8. Konzentrationen der erhaltenen Rohprodukte (Gew.-%) wurden GC-analytisch mittels internem Standard ermittelt.

Der Wassergehalt der erhaltenen Rohprodukte wurde mittels Karl-Fischer-Titration bestimmt.

### Beispiel 1:

Eine Apparatur bestehend aus einem Doppelmantelglasrohrreaktor mit einem Innendurchmesser von 2 cm und einer Länge von 36 cm wurde mit 50 g des stark sauren Kationenaustauschers Amberlyst™ 131 gefüllt. Der Kationenaustauscher wurde vor dem Einsatz zunächst mehrmals mit Wasser gewaschen, dann einmal mit Methanol und abschließend mit Wasser Methanol-frei gewaschen.

Der Doppelmantelglasreaktor wurde mit einem Gemisch aus Isovaleraldehyd (112,5 g, 1.31 mol), Isoprenol (125 g, 1.45 mol) und 12,5 g Wasser bei Raumtemperatur befüllt. Die Reaktionslösung wurde 8 h bei einer Temperatur von 40 °C mit einem Umpumpvolumen von 910 ml/h umgepumpt. Der Doppelmantelglasreaktor wurde mit einer Temperatur von 40°C betrieben. Man erhielt ein Rohprodukt in einer Menge von 250,5 g (Ausbeute 73%) mit der folgenden Zusammensetzung:

| | |
|---|---|
| Isovaleraldehyd: | 0,56 GC-Gew.%, |
| Isoprenol: | 2,35 GC-Gew.%, |
| Dihydropyran-Isomere (IVa - c): | 9,41 GC-FI% (GC-Flächen%) |
| 1,3-Dioxan (Va): | 10,25 GC-Gew.%, |
| Acetal (VIa): | 0,99 GC-FI%, |
| trans-Pyranol (le): | 17,49 GC-Gew.%, |
| cis-Pyranol (ld): | 48,28 GC-Gew.%. |
| Wasser: | 6,9% |

### Beispiel 2:

Der Doppelmantelglasreaktor wurde mit einem Gemisch aus Isovaleraldehyd (77.4 g, 0.9 mol), Isoprenol (86.1g, 1.0 mol) und 8.6 g Wasser bei Raumtemperatur befüllt. Die Reaktionslösung wurde 10 h bei einer Temperatur von 25 °C mit einem Umpumpvolumen von 1.5 l/h umgepumpt. Der Doppelmantelglasreaktor wurde auf 25°C temperiert. Man erhielt ein Rohprodukt in einer Menge von 169.4 g (Ausbeute 79%) mit der folgenden Zusammensetzung:

| | |
|---|---|
| Isovaleraldehyd: | 0,44 GC-FI.%, |
| Isoprenol: | 3,57 GC-FI.%, |
| Dihydropyran-Isomere (IVa - c): | 9,76 GC-FI.%, |
| 1,3-Dioxan (Va): | 3,16 GC-FI.%, |
| Acetal (VIa): | 0,99 GC-FI.%, |
| trans-Pyranol (Ie): | 18,91 GC-Gew.%, |
| cis-Pyranol (Id): | 54,13 GC-Gew.%. |
| Wasser | 6,9% |

## Patentansprüche

1. Verfahren zur Herstellung von 2-substituierten 4-Hydroxy-4-methyl-tetrahydropyranen der Formel (I) 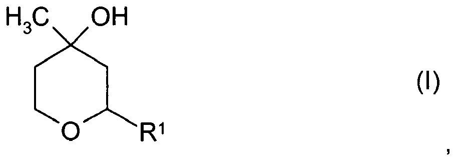 wobei der Rest
R¹ einen geradkettigen oder verzweigten Alkyl- oder Alkenylrest mit 1 bis 12 Kohlenstoffatomen, einen gegebenenfalls Alkyl-substituierten Cycloalkylrest mit insgesamt 3 bis 12 Kohlenstoffatomen oder einen gegebenenfalls Alkyl- und/oder Alkoxy-substituierten Arylrest mit insgesamt 6 bis 12 Kohlenstoffatomen bedeutet,
umfassend die Umsetzung von 3-Methylbut-3-en-1-ol der Formel (II) 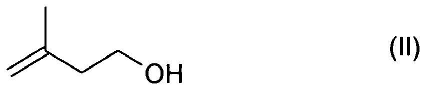 mit einem Aldehyd der Formel (III)
R¹-CHO (III),
wobei der Rest R¹ die gleiche Bedeutung wie in Formel (I) hat und
wobei man die Umsetzung in Gegenwart von Wasser und in Gegenwart eines stark sauren Kationenaustauschers durchführt.

2. Verfahren nach Anspruch 1, **dadurch gekennzeichnet, dass** der Rest
R¹ einen geradkettigen oder verzweigten Alkyl- oder Alkenylrest mit 1 bis 12 Kohlenstoffatomen, oder einen gegebenenfalls Alkyl- und/oder Alkoxy-substituierten Arylrest mit insgesamt 6 bis 12 Kohlenstoffatomen
bedeutet.

3. Verfahren nach Anspruch 1 oder 2, **dadurch gekennzeichnet, dass** der Rest R¹ Isobutyl bedeutet.

4. Verfahren nach Anspruch 1 oder 2, **dadurch gekennzeichnet, dass** der Rest R¹ Phenyl bedeutet.

5. Verfahren nach einem der Ansprüche 1 bis 4, **dadurch gekennzeichnet, dass** man Isoprenol und den Aldehyd der Formel (III) in einem molaren Verhältnis von 0,7 zu 1 bis 2 zu 1 einsetzt.

6. Verfahren nach Anspruch 5, **dadurch gekennzeichnet, dass** man Isoprenol und den Aldehyd der Formel (III) in einem molaren Verhältnis von 1 zu 1 bis 1,5 zu 1 einsetzt.

7. Verfahren nach einem der Ansprüche 1 bis 6, **dadurch gekennzeichnet, dass** man die Umsetzung in Gegenwart einer mindestens äquimolaren Menge Wasser durchführt, wobei sich die Menge an Wasser auf die Menge des gegebenenfalls im Unterschuss eingesetzten Ausgangsstoffes Isoprenol oder den Aldehyd der Formel (III), oder, in dem Fall der äquimolaren Umsetzung der beiden Ausgangsstoffe, auf die Stoffmenge eines der beiden bezieht.

8. Verfahren nach einem der Ansprüche 1 bis 7, **dadurch gekennzeichnet, dass** man einen stark sauren, Sulfonsäuregruppen umfassenden Kationenaustauscher einsetzt.

9. Verfahren nach einem der Ansprüche 1 bis 8, **dadurch gekennzeichnet, dass** man mindestens einen stark sauren Kationenaustauscher in der H(+)-Form einsetzt, wobei der lonenaustauscher ein Sulfonsäuregruppen aufweisendes Polymergerüst enthält und entweder gelförmig ist oder makroporöse Harze enthält.

10. Verfahren gemäss einem der Ansprüche 1 bis 9, **dadurch gekennzeichnet, dass** der lonenaustauscher auf einem Polystyrolgerüst mit Sulfonsäuregruppen oder auf einem perfluorierten Ionenaustauscherharz mit Sulfonsäuregruppen basiert.

11. Verfahren nach einem der Ansprüche 1 bis 10, **dadurch gekennzeichnet, dass** man die Umsetzung ohne Zusatz eines organischen Lösungsmittels durchführt.

12. Verfahren nach einem der Ansprüche 1 bis 11, **dadurch gekennzeichnet, dass** man die Umsetzung bei einer Temperatur im Bereich von 20 bis 60°C durchführt.

13. Verfahren nach einem der Ansprüche 1 bis 12, **dadurch gekennzeichnet, dass** man die Umsetzung kontinuierlich durchführt.

14. Verfahren nach Anspruch 13, umfassend die Schritte
a. Bereitstellen eines den gewählten stark sauren Kationenaustauscher enthaltenden Durchflussreaktors;
b. kontinuierliches Eintragen von Isoprenol, dem Aldehyd der Formel (III) sowie Wasser in den Durchflussreaktor;
c. kontinuierliches Inkontaktbringen von Isoprenol, dem Aldehyd der Formel (III) sowie Wasser mit dem stark sauren Kationenaustauscher im Durchflussreaktor unter Erhalt eines die gewünschten 2-substituierten 4-Hydroxy-4-methyl-tetrahydropyrane umfassenden Reaktionsgemisches und
d. kontinuierliches Austragen des Reaktionsgemisches aus dem Durchflussreaktor.

15. Verfahren nach einem der Ansprüche 1 bis 14 zur Herstellung von 2-substituierten 4-Hydroxy-4-methyl-tetrahydropyranen in Form von Gemischen der cis-Diastereomeren der Formeln (Ib) 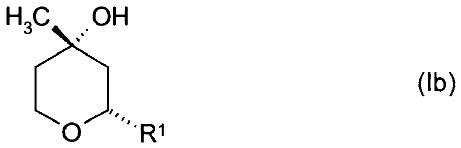 und der trans-Diastereomeren der Formel (Ic) 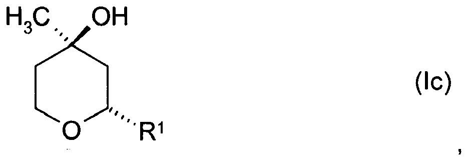 wobei das Diastereomerenverhältnis des cis-Diasteromeren der Formel (Ib) zum trans-Diastereomeren der Formel (Ic) 65 zu 35 bis 95 zu 5 beträgt, und R¹ die in den Ansprüchen 1, 2, 3 und 4 angegebenen Bedeutungen hat.

## Claims

1. A process for the preparation of 2-substituted 4-hydroxy-4-methyltetrahydropyrans of the formula (I) 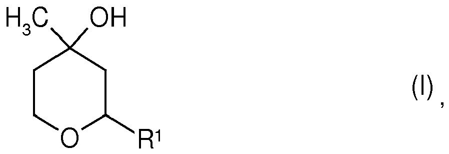 where the radical
R¹ is a straight-chain or branched alkyl or alkenyl radical having 1 to 12 carbon atoms, an optionally alkyl-substituted cycloalkyl radical having in total 3 to 12 carbon atoms or an optionally alkyl- and/or alkoxy-substituted aryl radical having in total 6 to 12 carbon atoms,
comprising the reaction of 3-methylbut-3-en-1-ol of the formula (II) 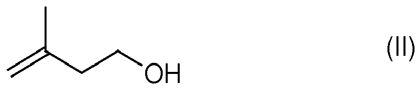 with an aldehyde of the formula (III)
R¹-CHO (III),
where the radical R¹ has the same meaning as in formula (I) and
where the reaction is carried out in the presence of water and in the presence of a strongly acidic cation exchanger.

2. The process according to claim 1, wherein the radical
R¹ is a straight-chain or branched alkyl or alkenyl radical having 1 to 12 carbon atoms, or an optionally alkyl- and/or alkoxy-substituted aryl radical having in total 6 to 12 carbon atoms.

3. The process according to claim 1 or 2, wherein the radical R¹ is isobutyl.

4. The process according to claim 1 or 2, wherein the radical R¹ is phenyl.

5. The process according to any one of claims 1 to 4, wherein isoprenol and the aldehyde of the formula (III) are used in a molar ratio of from 0.7:1 to 2:1.

6. The process according to claim 5, wherein isoprenol and the aldehyde of the formula (III) are used in a molar ratio of from 1:1 to 1.5:1.

7. The process according to any one of claims 1 to 6, wherein the reaction is carried out in the presence of an at least equimolar amount of water, where the amount of water refers to the amount of the starting material isoprenol, used optionally in deficit, or to the aldehyde of the formula (III), or, in the case of the equimolar reaction of the two starting materials, to the quantitative amount of one of the two.

8. The process according to any one of claims 1 to 7, wherein a strongly acidic cation exchanger comprising sulfonic acid groups is used.

9. The process according to any one of claims 1 to 8, wherein at least one strongly acidic cation exchanger in the H(+) form is used, where the ion exchanger comprises a polymer backbone having sulfonic acid groups and is either gel-like or comprises macroporous resins.

10. The process according to any of claims 1 to 9, wherein the ion exchanger is based on a polystyrene backbone with sulfonic acid groups or on a perfluorinated ion exchanger resin with sulfonic acid groups.

11. The process according to any one of claims 1 to 10, wherein the reaction is carried out without addition of an organic solvent.

12. The process according to any one of claims 1 to 11, wherein the reaction is carried out at a temperature in the range from 20 to 60°C.

13. The process according to any one of claims 1 to 12, wherein the reaction is carried out continuously.

14. The process according to claim 13, comprising the steps
a. providing a flow reactor comprising the selected strongly acidic cation exchanger;
b. continuously introducing isoprenol, the aldehyde of the formula (III) and water into the flow reactor;
c. continuously bringing isoprenol, the aldehyde of the formula (III) and water into contact with the strongly acidic cation exchanger in the flow reactor to give a reaction mixture comprising the desired 2-substituted 4-hydroxy-4-methyltetrahydropyrans and
d. continuously discharging the reaction mixture from the flow reactor.

15. The process according to any one of claims 1 to 14 for the preparation of 2-substituted 4-hydroxy-4-methyltetrahydropyrans in the form of mixtures of the cis-diastereomers of the formulae (Ib) 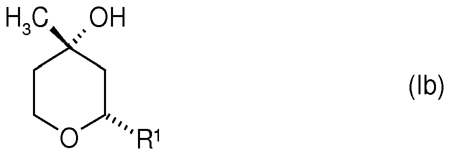 and of the trans-diastereomers of the formula (Ic) 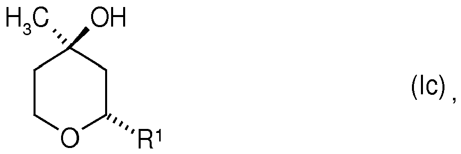 where the diastereomer ratio of the cis-diastereomer of the formula (Ib) to the trans-diastereomer of the formula (Ic) is 65:35 to 95:5, and R¹ has the meanings given in claims 1, 2, 3 and 4.

## Revendications

1. Procédé pour la préparation de 4-hydroxy-4-méthyltétrahydropyranes substitués en 2ème position de formule (I) 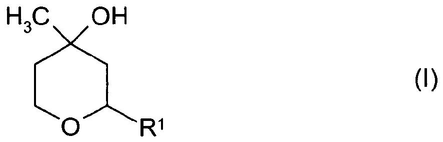 dans laquelle le radical
R¹ signifie un radical alkyle ou alcényle linéaire ou ramifié comprenant 1 à 12 atomes de carbone, un radical cycloalkyle le cas échéant substitué par alkyle présentant au total 3 à 12 atomes de carbone ou un radical aryle le cas échéant substitué par alkyle et/ou alcoxy présentant au total 6 à 12 atomes de carbone, comprenant la transformation de 3-méthylbut-3-én-1-ol de formule (II) 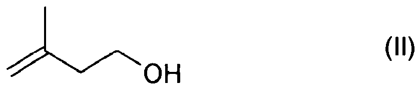 avec un aldéhyde de formule (III)
R¹- CHO (III),
le radical R¹ ayant la même signification que dans la formule (I) et
la transformation étant réalisée en présence d'eau et en présence d'un échangeur de cations fortement acide.

2. Procédé selon la revendication 1, **caractérisé en ce que** le radical
R¹ signifie un radical alkyle ou alcényle linéaire ou ramifié comprenant 1 à 12 atomes de carbone ou un radical aryle le cas échéant substitué par alkyle et/ou alcoxy présentant au total 6 à 12 atomes de carbone.

3. Procédé selon la revendication 1 ou 2, **caractérisé en ce que** le radical R¹ signifie isobutyle.

4. Procédé selon la revendication 1 ou 2, **caractérisé en ce que** le radical R¹ signifie phényle.

5. Procédé selon l'une quelconque des revendications 1 à 4, **caractérisé en ce qu'**on utilise l'isoprénol et l'aldéhyde de formule (III) dans un rapport molaire de 0,7:1 à 2:1.

6. Procédé selon la revendication 5, **caractérisé en ce qu'**on utilise l'isoprénol et l'aldéhyde de formule (III) dans un rapport molaire de 1:1 à 1,5:1.

7. Procédé selon l'une quelconque des revendications 1 à 6, **caractérisé en ce qu'**on réalise la transformation en présence d'une quantité au moins équimolaire d'eau, la quantité d'eau se rapportant à la quantité de la substance de départ, le cas échéant utilisée en une quantité inférieure à la quantité stoechiométrique, l'isoprénol ou l'aldéhyde de formule (III) ou, dans le cas d'une transformation équimolaire des deux substances, à la quantité d'une des deux substances.

8. Procédé selon l'une quelconque des revendications 1 à 7, **caractérisé en ce qu'**on utilise un échangeur de cations fortement acide, comprenant des groupes acide sulfonique.

9. Procédé selon l'une quelconque des revendications 1 à 8, **caractérisé en ce qu'**on utilise au moins un échangeur de cations fortement acide sous forme H(+), l'échangeur d'ions contenant une structure polymère présentant des groupes acide sulfonique et étant sous forme de gel ou contenant des résines macroporeuses.

10. Procédé selon l'une quelconque des revendications 1 à 9, **caractérisé en ce que** l'échangeur d'ions est à base d'une structure de polystyrène présentant des groupes acide sulfonique ou à base d'une résine échangeuse d'ions perfluorée présentant des groupes acide sulfonique.

11. Procédé selon l'une quelconque des revendications 1 à 10, **caractérisé en ce qu'**on réalise la transformation sans addition d'un solvant organique.

12. Procédé selon l'une quelconque des revendications 1 à 11, **caractérisé en ce qu'**on réalise la transformation à une température dans la plage de 20 à 60°C.

13. Procédé selon l'une quelconque des revendications 1 à 12, **caractérisé en ce qu'**on réalise la transformation en continu.

14. Procédé selon la revendication 13, comprenant les étapes
a. mise à disposition d'un réacteur à écoulement contenant l'échangeur de cations fortement acide choisi ;
b. introduction continue d'isoprénol, de l'aldéhyde de formule (III) ainsi que d'eau dans le réacteur à écoulement ;
c. mise en contact continue d'isoprénol, de l'aldéhyde de formule (III) ainsi que d'eau avec l'échangeur de cations fortement acide dans le réacteur à écoulement avec obtention d'un mélange réactionnel contenant le 4-hydroxy-4-méthyltétrahydropyrane substitué en 2ème position souhaité et
d. évacuation continue du mélange réactionnel du réacteur à écoulement.

15. Procédé selon l'une quelconque des revendications 1 à 14 pour la préparation de 4-hydroxy-4-méthyltétrahydropyranes substitués en 2ème position sous forme de mélanges des diastéréo-isomères cis des formules (Ib) 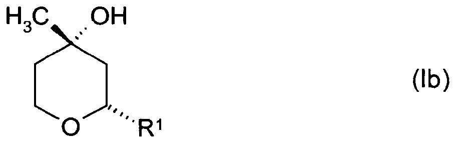 et des diastéréo-isomères trans de formule (Ic) 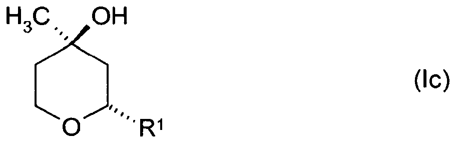 le rapport des diastéréo-isomères cis de formule (Ib) aux diastéréo-isomères trans de formule (Ic) valant 65:35 à 95:5, et R¹ ayant les significations indiquées dans les revendications 1, 2, 3 et 4.
